# EUROPÄISCHE PATENTSCHRIFT

(11) **EP 2 760 514 B1**
(45) Veröffentlichungstag und Bekanntmachung des Hinweises auf die Patenterteilung: **28.12.2016**
(21) Anmeldenummer: 12770077.1
(22) Anmeldetag: 27.09.2012
(51) Int. Cl.: G01J 5/02, A61M 16/16, G01J 5/00, G01J 5/08, A61M 16/10

(54) **ATEMLUFTBEFEUCHTER MIT EINER TEMPERATURMESSVORRICHTUNG**
RESPIRATORY HUMIDIFIER WITH A TEMPERATURE MEASURING DEVICE
HUMIDIFICATEUR D'AIR INHALÉ AVEC UN DISPOSITIF DE MESURE DE TEMPÉRATURE

(30) Priorität: 01.10.2011 DE 102011054132
(43) Veröffentlichungstag der Anmeldung: 06.08.2014
(73) Patentinhaber: Hamilton Bonaduz AG, 7402 Bonaduz (CH)
(72) Erfinder: BÜCHI, Rudolf, 7000 Chur (CH)
(74) Vertreter: Caspary, Karsten
(86) Internationale Anmeldenummer: PCT/EP2012/069117
(87) Internationale Veröffentlichungsnummer: WO 2013/045569

(56) Entgegenhaltungen:
- EP-A1- 0 411 121
- WO-A1-2004/020031
- DE-A1- 10 321 649
- DE-A1- 19 947 123
- DE-A1-102007 037 955
- DE-A1-102008 001 022
- DE-C1- 19 913 783
- US-A- 5 468 961
- US-A- 5 793 044
- US-A1- 2008 225 925

## Beschreibung

Die vorliegende Erfindung betrifft eine Temperaturmessvorrichtung für Atemluftbefeuchter beim Beatmen von Patienten mit Atemgas.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen üblicher Weise mit destilliertem Wasser gefüllten Flüssigkeitsbehälter auf, dessen Bodenplatte in thermischem Kontakt mit einer Heizplatte des Gehäuses steht, wodurch die Flüssigkeit erwärmt wird. Das Einatemgas wird in den Flüssigkeitsbehälter geleitet, mit Feuchtigkeit angereichert und verlässt den Flüssigkeitsbehälter mit einer bestimmten Temperatur.

Die Messung der Atemgastemperatur erfolgt zum einen üblicher Weise unter Verwendung eines nahe am Patienten angeordneten Temperatursensors, der mittels einer elektrischen Messleitung mit einer Steuereinrichtung, die beispielsweise im Atemluftbefeuchter angeordnet ist, verbunden ist. Zum anderen wird die Temperatur des Atemgases innerhalb des Atemluftbefeuchters, vorzugsweise beim Eingang in den und beim Ausgang aus dem Flüssigkeitsbehälter gemessen. Die gemessenen Werte werden einer im Atemluftbefeuchter befindlichen Steuereinrichtung zugeführt, die eine Regelung der Heizleistung des Atemluftbefeuchters vornimmt.

Pyrometer oder Strahlungsthermometer zur berührungslosen Temperaturmessung sind im Stand der Technik bekannt. Diese nutzen die Tatsache, dass jeder Körper mit einer Temperatur größer als 0 Kelvin eine Wärmestrahlung emittiert, deren Intensität und Lage des Emissionsmaximums von seiner Temperatur abhängt. Diese Strahlung wird mit dem Pyrometer erfasst und ausgewertet. Für Temperaturmessungen um die Raumtemperatur herum (ca. 10°C bis ca. 40°C) kommen Wellenlängen im Mittleren Infrarotbereich (MIR) in Frage. Die DE 10 2007 037 955 A1 offenbart eine berührungslose Temperaturmesseinrichtung für einen Beatmungsanfeuchter mit einem Strömungskanal, in dem ein geschlossener Hohl-9557 P 4981 EP körper für die Aufnahme der Temperatur in den Strömungskanal hinein ragt und bei dem ein Infrarotdetektor auf die Innenfläche des in den Strömungskanal hinein ragenden Hohlkörpers ausgerichtet ist. Nachteilig an diesem Aufbau der Temperaturmesseinrichtung ist, dass der Strömungskanal mit dem in ihn hinein ragenden Hohlkörper nicht einfach herzustellen ist, da der Hohlkörper entweder in einem zweiten Produktionsschritt in den Strömungskanal hinein geformt oder geklebt werden muss, oder alternativ die Herstellung mittels einer eigenen Form für Strömungskanal und Hohlkörper aufwendig und teuer ist. Darüber hinaus stellt der Hohlkörper im Strömungskanal einen relativ hohen Strömungswiderstand dar, der den Fluss des Atemgasstroms behindert und damit die Flussgeschwindigkeit reduziert. Die DE 10 2007 037 955 A1 offenbart einen Atemluftbefeuchter mit einer berührungslose Temperaturmesseinrichtung gemäß dem Oberbegriffs des Anspruchs 1.

Die DE 199 13 783 C1 offenbart ein Atemalkohol-Messgerät mit einem Probeentnahmekanal zur Aufnahme der Atemgasprobe und einem im Probeentnahmekanal angeordneten Temperaturfühler, der als infrarot-optisches Thermometer mit einem im Probeentnahmekanal befindlichen, für Infrarotstrahlung durchlässigen Filter ausgebildet ist.

Es ist daher die Aufgabe der vorliegenden Erfindung einen Atemluftbefeuchter mit einer Temperaturmessvorrichtung bereit zu stellen, die einfach und kostengünstig herzustellen ist und dennoch eine zuverlässige berührungslose Temperaturmessung ermöglicht.

Diese Aufgabe wird durch die Merkmale des Anspruchs 1 gelöst. Vorteilhafte Ausgestaltungen und Ausführungsformen sind in den Unteransprüchen beschrieben.

Erfindungsgemäß wird ein Atemluftbefeuchter mit den Merkmalen des Anspruchs 1 bereit gestellt. Die Anordnung des Messabschnitts auf der Mantelfläche des Strömungskanals stellt eine einfache Konstruktion dar, weil der Messabschnitt wie z.B. in der DE 10 2007 037 955 A1 nicht aus der Mantelfläche hervorragt. Als Mantelfläche im hier verwendeten Sinne ist beispielsweise die Wandfläche eines zylinderförmigen Strömungskanals oder eine ebene Wand eines Kanals mit rechteckigem oder polygonalem Querschnitt zu verstehen. Derartige Strömungskanäle können integral mit dem Flüssigkeitsbehälter ausgebildet sein und lassen sich einzeln oder zusammen mit dem Flüssigkeitsbehälter einfach und kostengünstig herstellen. Dabei ist der Messabschnitt flächig und quasi durchgängig mit den umgebenden Bereichen der Mantelfläche ausgerichtet bzw. schließt bündig damit ab.

Der Anströmwinkel beträgt bevorzugt ca. 30° bis ca. 60° und besonders bevorzugt ca. 40° bis ca. 50°. Ein mit diesem Anströmwinkel auf den Messabschnitt treffender Atemgasstrom wird direkt auf den Messabschnitt umgelenkt und gibt durch das Auftreffen auf den Messabschnitt einen Teil seiner Wärme besonders effektiv auf das Material des Messabschnitts ab. Die Temperatur des Messabschnitts entspricht dadurch noch besser im Wesentlichen der Temperatur des auftreffenden Atemgastroms.

Mit besonderem Vorteil ist das Strömungsleitelement im Inneren des Strömungskanals angeordnet. Dabei kann das Strömungsleitelement bevorzugt eine gerade oder eine gebogene Platte sein. Mit einem derartig ausgestalteten Strömungsleitelement kann ein beispielsweise in Längsrichtung des Strömungskanals eintreffender Atemgasstrom auf den bevorzugten Anströmwinkel umgelenkt werden. Ein derartiges Strömungsleitelement im Inneren des Strömungskanals ist auf relativ einfache Weise herzustellen.

Möglich ist jedoch auch, dass das Strömungsleitelement ein gerades oder gebogenes Rohr mit rechteckigem, kreisförmigem, polygonalem oder ellipsenförmigem Querschnitt ist.

Alternativ ist der Strömungskanal selbst so ausgebildet, dass er ein Strömungsleitelement aufweist. Dazu kann der Strömungskanal selbst derart gebogen sein oder einen Knick aufweisen, dass sich der gewünschte Anströmwinkel auf den Messabschnitt einstellt.

Mit weiterem Vorteil kann die Oberfläche des Strömungsleitelements strukturiert sein. Eine mögliche Struktur kann beispielsweise sein, dass die Oberfläche Löcher aufweist, aufgeraut ist, eine Mehrzahl von Erhebungen oder Ausnehmungen aufweist, die unterschiedlich geformt sein können, und dergleichen mehr. Die strukturierte Oberfläche dient der Erhöhung der Verwirbelung des Atemgasstroms, so dass dieser eine möglichst homogene Erwärmung des Messabschnitts bewirkt. Zusätzlich kann die Verwirbelung des Atemgasstroms für eine bessere Feuchtigkeits- und Wärmeaufnahme des Atemgases hilfreich sein.

Mit weiterem Vorteil ist der Messabschnitt dünnwandig ausgebildet, und das Material des Messabschnitts weist einen Emissionsgrad von etwa 0,90 bis etwa 1,00 auf. Die in Bezug auf die angrenzenden Mantelflächenabschnitte geringere Dicke bewirkt, dass sich der Messabschnitt schneller erwärmt und seine Temperatur besser, d.h. homogener, verteilt ist. Damit die Messung besonders gut ist, soll das Material thermisch optimiert sein, d. h. einen Emissionsgrad von nahe 1,00 aufweisen. Dafür kann der Messabschnitt mit einer schwarzen Schicht ausgestaltet sein, die beispielsweise aufgeklebt oder in anderer Form aufgebracht ist.

Bevorzugt weist der Infrarotdetektor thermische Infrarotmesselemente wie Bolometer, pyroelektrische Sensoren oder Thermosäulen (Thermopile) auf. Derartige Infrarotmesselemente sind für einen Temperaturbereich sehr gut geeignet, in dem Atemluftbefeuchter bevorzugt eingesetzt werden, d. h. in einem Umgebungstemperaturbereich von ca. 10° C bis ca. 40° C. Der Fachmann kann jedoch auch andere Infrarotmesselemente verwenden, die für den oben beschriebenen Temperaturbereich geeignet sind.

Zum Fokussieren kann der Infrarotdetektor bevorzugt ein optisches Bauelement wie z. B. eine Linse aufweisen. Mit weiterem Vorteil umfasst das Material des Strömungskanals bzw. des Flüssigkeitsbehälters einen transparenten Kunststoff wie z.B. LD-PE (Low Density Polyethylen). Transparent deshalb, weil ein Benutzer von außen erkennen können soll, ob Kondensation in dem Flüssigkeitsbehälter auftritt. Andere geeignete transparente Kunststoffmaterialien können ebenfalls verwendet werden.

Mit Vorteil weist der Atemluftbefeuchter eine Steuereinrichtung auf, die dazu eingerichtet ist, die Heizleistung entsprechend dem Messwert der Temperaturmessvorrichtung zu regeln. Es versteht sich, dass im Falle von zwei Temperaturmessvorrichtungen die zweite den nach oben ausgeleiteten Atemgasstrom misst, der folglich von unten, d. h. von der Flüssigkeitsoberfläche in den Flüssigkeitsbehälter kommend, auf den zweiten Messabschnitt geleitet wird, bevor er über ein Schlauchverbindungselement in den Inspirationsschlauch hin zum Patienten austritt.

Die Erfindung soll nachfolgend anhand eines bevorzugten Ausführungsbeispiels unter Bezugnahme auf die beigefügten Figuren erläutert werden, in denen:
- Fig. 1: eine perspektivische und teilweise ausgeschnittene Ansicht eines erfindungsgemäßen Atemluftbefeuchters zeigt, der eine bevorzugte Ausführungsform der Temperaturmessvorrichtung aufweist;
- Fig. 2: eine Seitenansicht der wesentlichen Elemente der in Fig. 1 dargestellten bevorzugten Ausführungsform der vorliegenden Erfindung zeigt.

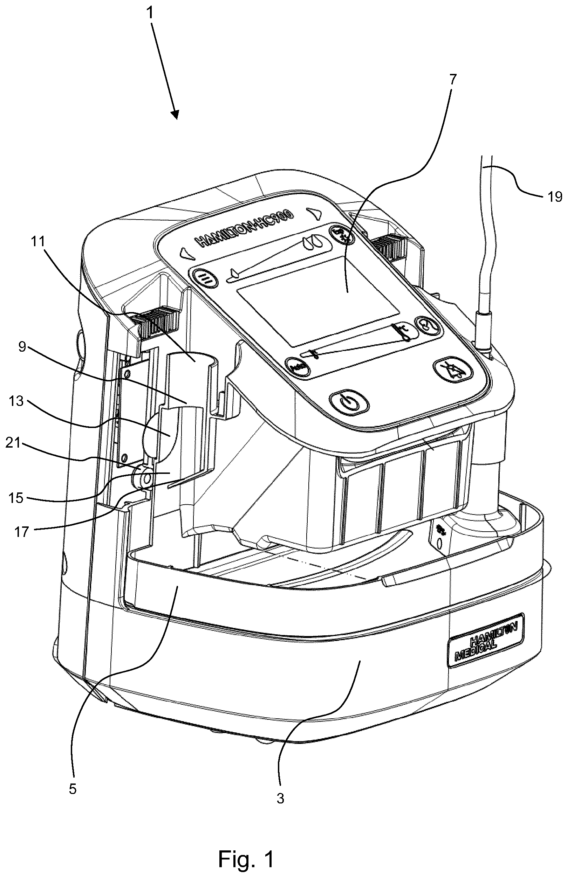

Fig. 1 zeigt in perspektivischer Ansicht einen Atemluftbefeuchter 1 mit einem Gehäuse 3 sowie einen auf das Gehäuse 3 lösbar aufgesteckten Flüssigkeitsbehälter 5, wobei zum besseren Verständnis der Erfindung ein Teil des Gehäuses 3 und der Flüssigkeitsbehälter 5 teilweise ausgeschnitten dargestellt sind. Der Flüssigkeitsbehälter 5 weist in horizontaler Richtung einen im Wesentlichen U-förmigen Querschnitt auf, so dass er nach dem seitlichen Aufstecken auf das Gehäuse 3 den zentralen, vorsprungartigen Abschnitt des Gehäuses 3, auf dem oben die Benutzerschnittstelle 7 mit Anzeige- und Bedienelementen angeordnet ist, umschließt. Im in Fig. 1 links dargestellten Bereich des Flüssigkeitsbehälters 5 weist dieser einen Strömungskanal 9 auf, der bei einem Rohrstutzen 11 mit kreisförmigem Querschnitt beginnt und sich von dort nach unten mit einer Mantelfläche 13 erstreckt. Die Mantelfläche 13, die als den Strömungskanal 9 flächig umgebende Wandfläche definiert ist, weist einen Messabschnitt 15 auf, dessen Wanddicke bezogen auf den Rest der Mantelfläche 13 dünner ausgebildet ist. Der Messabschnitt 15 ist mit den umgebenden Bereichen der Mantelfläche 13 ausgerichtet bzw. schließt damit bündig ab, ohne dass ein Element aus der Mantelfläche 13 in das Innere des Strömungskanals 9 hineinragt. Der Strömungskanal 9 weist in der hier dargestellten Ausführungsform ein Strömungsleitelement 17 auf, das sich als im Wesentlichen ebene Platte von dem der Mantelfläche 13 gegenüber liegenden Mantelflächenabschnitt in einem Winkel von ca. 60° bezogen auf den Messabschnitt 15 über etwa dreiviertel des Querschnitts des Strömungskanals 9 erstreckt.

Der hier dargestellte Flüssigkeitsbehälter 5 ist aus einem transparenten Kunststoffmaterial gebildet, so dass die Flüssigkeit darin und etwaige Kondensationsansammlungen von außerhalb sichtbar sind. Weiterhin weist der Flüssigkeitsbehälter 5 einen sich nach oben erstreckenden Nachfüllschlauch 19 auf, über den die Flüssigkeit in dem Flüssigkeitsbehälter 5 automatisch aus einem externen Reservoir nachgefüllt werden kann. Das Gehäuse 3 weist in seinem linken Bereich eine (in Fig. 1 teilweise ausgeschnittene) Wand auf, die im Betriebszustand parallel zur Mantelfläche 13 des Flüssigkeitsbehälters 5 angeordnet ist, wobei sich lediglich ein schmaler Luftspalt zwischen den beiden Flächen befindet. In dieser Wand des Gehäuses ist in einer Ausnehmung ein Infrarotdetektor 21 angeordnet, dessen Optik auf den Messabschnitt 15 der Mantelfläche 13 gerichtet ist.

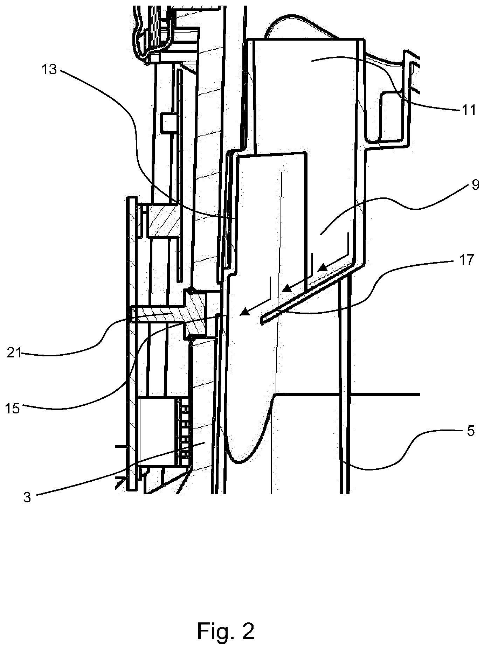

Fig. 2 zeigt eine seitliche Querschnittsansicht der Temperaturmessvorrichtung in der bevorzugten Ausführungsform aus Fig. 1. Der Ausschnitt der seitlichen Querschnittsansicht ist vergrößert und verzichtet auf die Darstellung der restlichen Elemente des Atemluftbefeuchters, die für die vorliegende Erfindung nicht wesentlich sind. Deutlich ist zu sehen, wie das Strömungsleitelement 17 den über den Rohrstutzen 11 einströmenden Atemgasstrom im Strömungskanal 9 auf den Messabschnitt 15 umlenkt. Die drei Pfeile geben prinzipiell die Strömungsrichtung des Atemgasstroms am Strömungsleitelement 17 an.

Im linken Abschnitt von Fig. 2 ist der schraffiert dargestellte Infrarotdetektor 21 deutlich erkennbar, der von außerhalb des Flüssigkeitsbehälters 5 waagerecht auf den Messabschnitt 15 gerichtet ist. Der Infrarotdetektor 21 ist mit einer (nicht dargestellten) Steuereinrichtung verbunden, die am Gehäuse 3 zur Auswertung des gemessen Signals und zu entsprechenden Regelung der Temperatur bzw. der Heizleistung angeordnet ist.

Die oben beschriebene bevorzugte Ausführungsform stellt einen einfachen Lösungsansatz zur berührungslosen Temperaturmessung des Atemgasstroms im Flüssigkeitsbehälter 5 des Atemluftbefeuchters 1 dar, weil der Messabschnitt 15 auf einfache Weise in der Mantelfläche 13 des Strömungskanals 9 ausgebildet ist und auch das Strömungsleitelement 17 in einfacher Weise innerhalb des Strömungskanals 9 ausgebildet sein kann. Es ist darüber hinaus denkbar, das Strömungsleitelement 17 mehrteilig, gekrümmt, strukturiert oder mit einen anderen Anströmwinkel üblich des Messabschnitts 15 zu versehen, um so eine optimierte Anströmung und damit Temperaturverteilung auf dem Messabschnitt 15 zu erzielen.

Mit dem Gegenstand der vorliegenden Erfindung wurde ein Atemluftbefeuchter mit einer Temperaturmessvorrichtung bereit gestellt, die einfach und kostengünstig herzustellen ist und die dennoch eine zuverlässige berührungslose Temperaturmessung ermöglicht.

## Patentansprüche

1. Atemluftbefeuchter (1) mit einem Gehäuse (3), einem lösbar aufgesteckten Flüssigkeitsbehälter (5) und einer Temperaturmessvorrichtung, wobei die Temperaturmessvorrichtung am Flüssigkeitsbehälter (5) einen Strömungskanal (9) für Atemgas und am Gehäuse (3) einen Infrarotdetektor (21) aufweist, der zur berührungslosen Erfassung der Temperatur des Atemgases im Strömungskanal (9) von außen auf den Strömungskanal (9) gerichtet ist,
**dadurch gekennzeichnet, dass**
der Strömungskanal (9) auf seiner Mantelfläche (13), die als den Strömungskanal (9) flächig umgebende Wandfläche definiert ist, einen Messabschnitt (15) aufweist, der durchgängig mit den umgebenden Bereichen der Mantelfläche (13) ausgerichtet ist und derart damit bündig abschließt, ohne dass ein Element aus der Mantelfläche (13) in das Innere des Strömungskanals (9) hineinragt, und auf den der Infrarotdetektor (21) gerichtet ist,
wobei der Strömungskanal (9) ein Strömungsleitelement (17) aufweist, das den Atemgasstrom in einem vorbestimmten Anströmwinkel, der vorzugsweise größer als 10° und kleiner als 170° ist, auf den Messabschnitt (15) umlenkt.

2. Atemluftbefeuchter (1) nach Anspruch 1, **dadurch gekennzeichnet, dass** der Anströmwinkel ca. 30° bis ca. 60° und besonders bevorzugt ca. 40° bis ca. 50° beträgt.

3. Atemluftbefeuchter (1) nach Anspruch 1 oder 2, **dadurch gekennzeichnet, dass** das Strömungsleitelement (17) im Inneren des Strömungskanals (9) angeordnet ist.

4. Atemluftbefeuchter (1) nach Anspruch 3, **dadurch gekennzeichnet, dass** das Strömungsleitelement (17) eine gerade oder gebogene Platte ist.

5. Atemluftbefeuchter (1) nach einem der vorhergehenden Ansprüche, **dadurch gekennzeichnet, dass** das Strömungsleitelement (17) ein gerades oder gebogenes Rohr mit rechteckigem, kreisförmigem, polygonalem oder ellipsenförmigem Querschnitt ist.

6. Atemluftbefeuchter (1) nach einem der Ansprüche 2 bis 5, **dadurch gekennzeichnet, dass** das Strömungsleitelement (17) eine strukturierte Oberfläche aufweist.

7. Atemluftbefeuchter (1) nach einem der vorhergehenden Ansprüche, **dadurch gekennzeichnet, dass** der Messabschnitt (15) dünnwandig ausgebildet ist und einen Emissionsgrad von etwa 0,90 bis etwa 1,00 aufweist.

8. Atemluftbefeuchter (1) nach einem der vorhergehenden Ansprüche, **dadurch gekennzeichnet, dass** der Infrarotdetektor (21) thermische Infrarotmesselemente wie Bolometer, pyroelektrische Sensoren oder Thermosäulen aufweist.

9. Atemluftbefeuchter (1) nach einem der vorhergehenden Ansprüche, **dadurch gekennzeichnet, dass** der Infrarotdetektor (21) zum Fokussieren ein optisches Bauelement aufweist.

10. Atemluftbefeuchter (1) nach einem der vorhergehenden Ansprüche, **dadurch gekennzeichnet, dass** das Material des Strömungskanals (9) ein transparenter Kunststoff ist.

11. Atemluftbefeuchter (1) nach einem der vorgehenden Ansprüche, **dadurch gekennzeichnet, dass** er eine Steuereinrichtung aufweist, die dazu eingerichtet ist, eine Heizleistung des Atemluftbefeuchters entsprechend dem Messwert der Temperaturmessvorrichtung zu regeln.

## Claims

1. Respiratory humidifier (1) with a housing (3), a detachably fitted fluid container (5) and a temperature measuring device, wherein the temperature measuring device on the fluid container (5) has a flow channel (9) for respiratory gas and on the housing (3) an infrared detector (21) which is directed from outside to the flow channel (9) for contactless detection of the temperature of the respiratory gas in the flow channel (9),
**characterised in that** the flow channel (9) has on its lateral surface (13) which is designed as the wall face surrounding the surface area of the flow channel (9), a measuring portion (15) which is continuously aligned with the surrounding regions of the lateral surface (13) and closes flush therewith without an element projecting from the lateral surface (13) into the interior of the flow channel (9), and to which the infrared detector (21) is directed,
wherein the flow channel (9) has a flow guide element (17) which diverts the respiratory gas stream to the measuring portion (15) at a predetermined inflow angle which is preferably greater than 10° and smaller than 170°.

2. Respiratory humidifier (1) according to claim 1, **characterised in that** the inflow angle amounts to about 30° to about 60° and more particularly preferred to about 40° to about 50°.

3. Respiratory humidifier (1) according to claim 1 or 2 **characterised in that** the flow guide element (17) is arranged inside the flow channel (9).

4. Respiratory humidifier (1) according to claim 3, **characterised in that** the flow guide element (17) is a straight or bent plate.

5. Respiratory humidifier (1) according to one of the preceding claims, **characterised in that** the flow guide element (17) is a straight or bent tube with rectangular, circular, polygonal or elliptical cross-section.

6. Respiratory humidifier (1) according to one of claims 2 to 5 **characterised in that** the flow guide element (17) has a structured surface.

7. Respiratory humidifier (1) according to one of the preceding claims, **characterised in that** the measuring portion (15) is configured with thin walls and has an emissions degree of about 0.90 to about 1.00.

8. Respiratory humidifier (1) according to one of the preceding claims, **characterised in that** the infrared detector (21) has thermal infrared measuring elements such as bolometers, pyroelectric sensors or thermopiles.

9. Respiratory humidifier (1) according to one of the preceding claims, characterised the infrared detector (21) has an optical component for focussing.

10. Respiratory humidifier (1) according to one of the preceding claims, **characterised in that** the material of the flow channel (9) is transparent plastics.

11. Respiratory humidifier (1) according to one of the preceding claims, **characterised in that** it has a control unit which is set up to regulate the heating power of the respiratory humidifier corresponding to the measured value of the temperature measuring device.

## Revendications

1. Humidificateur d'air inhalé (1) comprenant un boîtier (3), un récipient de fluide (5) emboîté de manière amovible et un dispositif de mesure de température, dans lequel le dispositif de mesure de température présente sur le récipient de fluide (5) un canal d'écoulement (9) pour le gaz inhalé et sur le boîtier (3) un détecteur infrarouge (21), qui est dirigé depuis l'extérieur sur le canal d'écoulement (9) afin de détecter sans contact la température du gaz inhalé dans le canal d'écoulement (9),
**caractérisé en ce que**
le canal d'écoulement (9) présente, sur sa surface d'enveloppe (13), qui est définie comme la surface de paroi entourant à plat le canal d'écoulement (9), un tronçon de mesure (15), qui est aligné en continu avec les zones environnantes de la surface d'enveloppe (13) et qui se termine par ces dernières de manière affleurante sans qu'un élément ne dépasse de la surface d'enveloppe (13) à l'intérieur du canal d'écoulement (9), et sur lequel le détecteur infrarouge (21) est dirigé,
dans lequel le canal d'écoulement (9) présente un élément de guidage d'écoulement (17), qui dévie sur le tronçon de mesure (15) le flux de gaz inhalé selon un angle de flux entrant prédéfini, qui est de préférence supérieur à 10° et inférieur à 170°.

2. Humidificateur d'air inhalé (1) selon la revendication 1, **caractérisé en ce que** l'angle de flux entrant s'élève d'environ 30° à environ 60°, et de manière particulièrement préférée d'environ 40° à environ 50°.

3. Humidificateur d'air inhalé (1) selon la revendication 1 ou 2, **caractérisé en ce que** l'élément de guidage d'écoulement (17) est disposé à l'intérieur du canal d'écoulement (9).

4. Humidificateur d'air inhalé (1) selon la revendication 3, **caractérisé en ce que** l'élément de guidage d'écoulement (17) est une plaque droite ou cintrée.

5. Humidificateur d'air inhalé (1) selon l'une quelconque des revendications précédentes, **caractérisé en ce que** l'élément de guidage d'écoulement (17) est un tuyau droit ou cintré avec une section transversale rectangulaire, à forme circulaire, polygonale ou à forme elliptique.

6. Humidificateur d'air inhalé (1) selon l'une quelconque des revendications 2 à 5, **caractérisé en ce que** l'élément de guidage d'écoulement (17) présente une surface structurée.

7. Humidificateur d'air inhalé (1) selon l'une quelconque des revendications précédentes, **caractérisé en ce que** le tronçon de mesure (15) est réalisé de manière à présenter des parois minces et présente un degré d'émission d'environ 0,90 à environ 1,00.

8. Humidificateur d'air inhalé (1) selon l'une quelconque des revendications précédentes, **caractérisé en ce que** le détecteur infrarouge (21) présente des éléments de mesure infrarouges thermiques tels qu'un bolomètre, des capteurs pyroélectriques ou des thermopiles.

9. Humidificateur d'air inhalé (1) selon l'une quelconque des revendications précédentes, **caractérisé en ce que** le détecteur infrarouge (21) présente un élément structurel optique aux fins de la focalisation.

10. Humidificateur d'air inhalé (1) selon l'une quelconque des revendications précédentes, **caractérisé en ce que** le matériau du canal d'écoulement (9) est une matière plastique transparente.

11. Humidificateur d'air inhalé (1) selon l'une quelconque des revendications précédentes, **caractérisé en ce qu'**il présente un système de commande, qui est installé afin de régler une puissance de chauffage de l'humidificateur d'air inhalé selon la valeur de mesure du dispositif de mesure de température.
